# EUROPEAN PATENT APPLICATION

(11) **EP 1 384 783 A1**
(43) Date of publication of application: **28.01.2004**
(21) Application number: 02722902.0
(22) Date of filing: 30.04.2002
(51) Int. Cl.: C12N 15/55, C12N 9/22, C12Q 1/34, A61K 48/00, A61K 38/43

(54) **NOVEL MAXIZYME**

(30) Priority: 01.05.2001 JP 2001134469
(71) Applicant: National Institute of Advanced Industrial Science and Technology, Tokyo 100-8921 (JP); TAISHO PHARMACEUTICAL CO., LTD, Tokyo 170-8633 (JP); Genofunction, Inc., Tsukuba-shi, Ibaraki 305-0856 (JP)
(72) Inventor: TAIRA, K, c/o TSUKUBA CENTER, Tsubuka-shi, Ibaraki 305-8562 (JP); WARASHINA, Tomoko, c/o TSUKUBA CENTER, Tsubuka-shi, Ibaraki 305-8562 (JP); WARASHINA, Masaki, c/o TSUKUBA CENTER, Tsubuka-shi, Ibaraki 305-8562 (JP); KAWASAKI, Hiroaki, c/o TSUKUBA CENTER, Tsubuka-shi, Ibaraki 305-8562 (JP); HARA, T., c/o TAISHO PHARMACEUTICAL CO., LTD., Tokyo 170-8633 (JP); NOZAWA, Iwao, c/o GENOFUNCTION, INC., Tsukuba-shi, Ibaraki 305-0856 (JP)
(74) Representative: Sternagel, Fleischer, Godemeyer & Partner Patentanwälte
(86) International application number: PCT/JP2002/004322
(87) International publication number: WO 2002/092821

(57) **Abstract**

An object of the present invention is to provide a maxizyme that can bind to a target mRNA regardless of its conformation, and can effectively cleave the mRNA. The present invention provides a maxizyme which binds to a molecule having helicase activity.

SEQUENCE LISTING
<110> NATIONAL INSTITUTE OF ADVANCED INDUSTRIAL SCIENCE AND TECHNOLOGY
<110> TAISHO PHARMACEUTICAL CO.,LTD.
<110> GENOFUNCTION INC.
<120> Novel Maxizyme
<130> A21216A
<160> 8
<210> 1
<211>173
<212>RNA
<213>Homo Sapiens
<400>1
agaccaccuc cccugcgagc uaagcuggac agccaaugac ggguaagaga gugacauugu 60
ucacuaaccu aagacaggag ggccgucaga gcuacugccu aauccaaaga cggguaaaag 120
ugauaaaaau guaucacucc aaccuaagac aggcgcagcu uccgagggat ttg 173
<210>2
<211>60
<212>RNA
<213>Homo Sapiens
<400>2
gggucucucu gguuagacca gaucugagcc ugggagcucu cuggcuaacu agggaaccca 60
<210>3
<211>87
<212>RNA
<213>Homo Sapiens
<400>3
accguugguu uccguagugu agugguuauc acguucgccu aacacgcgaa agguccccgg 60

uucgaaaccg ggcacuacaa aaaccaac
<210>4
<211>30
<212>RNA
<213>Artificial Sequence
<400>4
aucuggucuc ugaugagcga aaccagagag 30
<210>5
<211>30
<212>RNA
<213>Artificial Sequence
<400>5
uauuccgcgc ugaugagcga aaccagagag 30
<210>6
<211>30
<212>RNA
<213>Artificial Sequence
<400>6
aucuggucuc ugaugagcga aacuugaugu 30
<210>7
<211>30
<212>RNA
<213>Artificial Sequence
<400>7
uauuccgcgc ugaugagcga aacuugaugu 30
<210>8
<211>30

<212>RNA

<213>Artificial Sequence

<400>8

gggucucucu gguuagacca gaucugagcc ugggagcucu cuggcuaacu agggaaccca 60

cugcuuaagc cucaauaaag cuuggcauuc cgguacuguu gguaaaaugg aagacgccaa 120

aaacauaaag aaaggcccgg cgccauucua uccucuagag gauggaaccg cuggagagca 180

acugcauaag gcuaugaaga gauacgcccu gguuccugga acaauugcuu uuacagaugc 240

acauaucgag gugaacauca cguacgcgga auacuucgaa auguccguuc gguuggcaga 300

## Description

### TECHNICAL FIELD

The present invention relates to a maxizyme and a use thereof, in particular to a maxizyme showing a good RNA-cleaving activity on a target RNA, and a use thereof.

### BACKGROUND ART

In early 1980's, the discovery of self-splicing of rRNA of Tetrahymena pyriformis by Cech et al., Colorado University, United States (K. Kruger, Cell, 31, 147-157 (1982)) and the analysis of ribonuclease P, a complex enzyme composed of RNA and protein, by Altman, Yale University, United States (C. Guerrier-Takada, Cell, 35, 849-857 (1983)) led to the discovery of the ribozyme (ribozyme: nucleotide acid + enzyme), which is a RNA having a catalytic function.

Since then, various ribozymes have been found. Among all, hammerhead ribozymes are one of the most well researched ribozymes. The hammerhead ribozyme, which functions to provide a self-cleavage reaction (cis-type) in nature was divided (converted to trans-type) into two RNA strands (a substrate region and an enzymatic activity retaining region) by the groups of Uhlenbeck et al. and Haseloff et al. (O.C. Uhlenbeck, Nature, 328, 596 (1987); J. Hasehoff, W.L. Gerlach, Nature, 334, 585 (1988)), whereby an application of a ribozyme for genetic therapy was suggested.

Since then, numerous forms of applied research targeting cancers, AIDS and the like have been reported (M. Cotten, M.L. Bimstlel, EMBO J 8, 861 (1989)). The present inventors have developed a maxizyme having an extremely high substrate-specificity and cleaving only mRNA having an abnormal junction sequence without affecting normal mRNA at all (International Publication: WO99/46388).

However, a target mRNA has a secondary structure or a tertiary structure. Thus, there has been a problem in that the substrate-binding region of the maxizyme hidden in the stem structure or the like may block the maxizyme from approaching its target site, so that no sufficient effect can be obtained.

### DISCLOSURE OF THE INVENTION

An object of the present invention is to provide a maxizyme that can bind to a target mRNA regardless of its conformation, and can effectively cleave the mRNA.

As a result of intensive studies to achieve the above object, the present inventors have found that excellent RNA cleaving activity can be obtained by allowing the maxizyme to act with a molecule having helicase activity, thereby completing the present invention.

The present invention relates to the following (1) to (19).
(1) A maxizyme which binds to a molecule having helicase activity.
(2) A maxizyme which comprises a region which binds to the molecule having helicase activity.
(3) A maxizyme which is composed of a dimeric structure formed by an RNA molecule containing the following nucleotide sequence (a) and an RNA molecule containing the following nucleotide sequence (b),

   5' X¹ ₗ...X¹ ₕ Y¹ ₗ...Y¹ ᵢZ¹ ₗ...Z¹ ⱼB¹ ₗ...B¹ ₚ 3' (a)

   5' Z² ₗ...Z² ₙ Y² ₗ...Y² ₘX² ₗ...X² ₖB² ₗ...B² _{q} 3' (b)

   wherein X¹ₗ...X¹ₕ, X²ₗ...X²ₖ, Y¹ₗ...Y¹ᵢ, Y²ₗ...Y²ₘ, Z¹ₗ...Z¹ⱼ, Z²ₗ...Z²ₙ, B¹ₗ...B¹ₚ and B²ₗ...B²_{q} are independently any one of A, U, T, C and G; and h and k are integers of 1 or more (for example, integers between 1 and 100);
   i and m are integers of 1 or more (for example, integers between 1 and 100);
   j and n are integers of 1 or more (for example, integers between 1 and 100);
   p and q are integers of 1 or more (for example, integers between 1 and 100);
   X¹ₗ...X¹ₕ and X²ₗ...X²ₖ are nucleotide sequences complementary to a specific sequence in a target RNA, or nucleotide sequences containing a region complementary to a sequence near a cleavage site of the target RNA and a region capable of forming a cavity for capturing Mg²⁺ (magnesium ion) only in the presence of the target RNA;
   Y¹ₗ...Y¹ᵢ and Y²ₗ...Y²ₘ are nucleotide sequences forming stems; and
   Z¹ₗ...Z¹ⱼ and Z²ₗ...Z²ₙ are nucleotide sequences containing a region complementary to a sequence near a cleavage site of the target RNA and a region capable of forming a cavity for capturing Mg²⁺ only in the presence of the target RNA, and one of or both B¹ₗ...B¹ₚ and B²ₗ...B²_{q} are nucleotide sequences containing a region that binds to a molecule having helicase activity.
(4) The maxizyme according to (3), wherein, as a nucleotide sequence capable of forming a cavity for capturing Mg²⁺ (magnesium ion) only in the presence of a target RNA, Z¹ₗ...Z¹ⱼ contains a sequence GAA, and Z²ₗ...Z²ₙ contains a sequence CUGAY_{Z}GA wherein Y_{Z} represents any one mononucleotide of A, G, U or C.
(5) The maxizyme according to (3) or (4), which contains, as a nucleotide sequence capable of forming a cavity for capturing Mg²⁺ only in the presence of a target RNA, a sequence GAA of X²ₗ...X²ₖ and a sequence CUGAY_{X}GA of X¹ₗ...X¹ₕ wherein Y_{X} represents any one mononucleotide of A, G, U or C.
(6) The maxizyme according to any one of (1) to (5), wherein the molecule having helicase activity is an RNA helicase.
(7) The maxizyme according to (6), wherein the region that binds to a molecule having helicase activity is nucleotides represented by SEQ ID NO: 1 (CTE).
(8) The maxizyme according to (6), wherein the region that binds to a molecule having helicase activity is nucleotides represented by SEQ ID NO: 2 (TAR).
(9) The maxizyme according to any one of (3) to (8), wherein a linker sequence and a tRNA^{val} promoter sequence are added upstream of each of the nucleotide sequences (a) and (b).
(10) The maxizyme according to (9), wherein the linker sequence added upstream of the nucleotide sequence (a) contains the following nucleotide sequence (e), and the linker sequence added upstream of the nucleotide sequence (b) contains the following nucleotide sequence (f):

   5' AAA 3' (e)

   5' UUU 3' (f)
(11) The maxizyme according to (9), wherein the tRNA^{val} promoter sequence added upstream of each of the nucleotide sequences (a) and (b) is SEQ ID NO: 3.
(12) The maxizyme according to (9), wherein an additional sequence and a terminator sequence are added downstream of each of the nucleotide sequences (a) and (b).
(13) The maxizyme according to (12), wherein the additional sequence added downstream of the nucleotide sequence (a) contains the following nucleotide sequence (g), the additional sequence added downstream of the nucleotide sequence (b) contains the following nucleotide sequence (h), and the terminator sequence added downstream of each of the nucleotide sequences (a) and (b) contains the following nucleotide sequence (i):

   5' AAA 3' (g)

   5' AACCGUA 3' (h)

   5' UUUUU 3' (i)
(14) An expression vector which contains a DNA encoding the maxizyme according to any one of (1) to (13).
(15) A complex which is composed of the maxizyme according to any one of (1) to (13) and a molecule having helicase activity.
(16) A pharmaceutical composition, which comprise the maxizyme according to any one of (1) to (13), the expression vector according to (14), or the complex according to (15) as an active ingredient.
(17) A method for cleaving a target nucleic acid by using the maxizyme according to any one of (1) to (13), the expression vector according to (14), or the complex according to (15).
(18) A method for specifically inhibiting or suppressing the biological functions of a target nucleic acid by using the maxizyme according to any one of (1) to (13), the expression vector according to (14), or the complex according to (15).
(19) A method for analyzing the biological functions of a target nucleic acid, which comprises specifically cleaving the target nucleic acid or specifically inhibiting the biological functions of the target nucleic acid by using the maxizyme according to any one of (1) to (13), the expression vector according to (14), or the complex according to (15); and examining the effect of the cleavage or the inhibition on the biological activities.

### BRIEF DESCRIPTION OF THE DRAWINGS

Fig. 1 shows the secondary structure of CTE (Constitutive transport element).
Fig. 2 shows a schematic diagram of CTE-Mz.

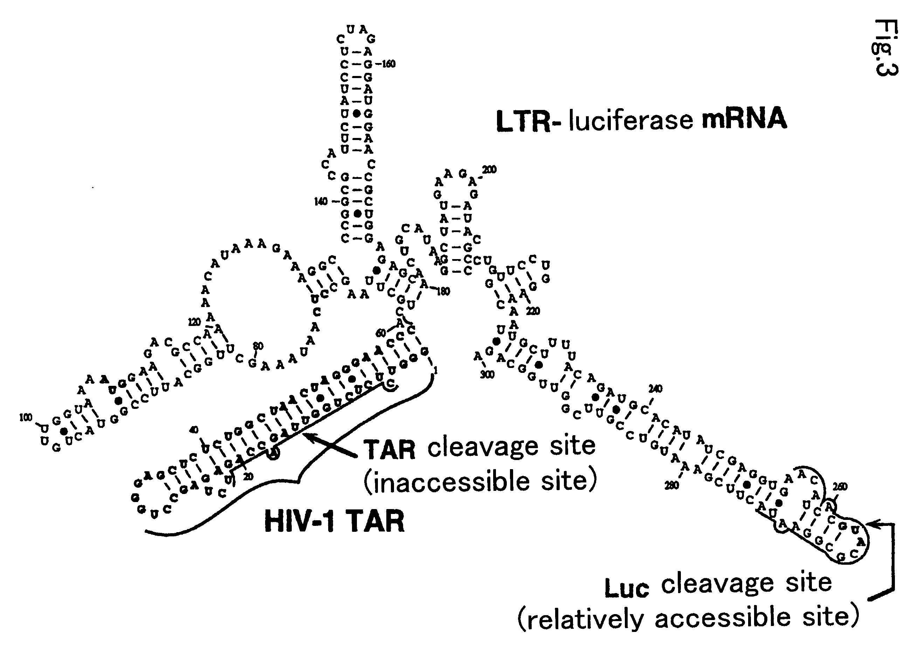

Fig. 3 shows the secondary structure of LTR-luciferase mRNA.
Fig. 4 shows Luc homo Mz which is bound to the Luc cleavage sites of two mRNAs.
Fig. 5 shows Luc homo Mz which is bound to the TAR cleavage sites of two mRNAs.
Fig. 6 shows Luc homo Mz which is bound to the Luc cleavage site and the TAR cleavage site of one mRNA.
Fig. 7 shows the maxizyme and the CTE-maxizyme which are bound to one LTR-luciferase mRNA (cis-type maxizyme).
Fig. 8 shows the maxizyme and the CTE-maxizyme which are bound to two LTR-luciferase mRNAs (trans-type maxizyme).
Fig. 9 shows the result of luciferase activity (homodimer type maxizyme).
Fig. 10 shows the result of luciferase activity (heterodimer type maxizyme).

### BEST MODE OF CARRYING OUT THE INVENTION

The maxizyme of the present invention is characterized by binding to a molecule having helicase activity.

The present inventors have assumed at first that when a molecule having helicase activity binds to the maxizyme, dimer formation is impossible due to the steric hindrance resulting from the binding. Particularly, when pol III system is used as an expression system, tRNA^{val} is added to the 5' end, and therefore steric hindrance of the maxizyme will be even greater. However surprisingly, it has been confirmed that the maxizyme forms a dimer and has RNA-cleaving activity superior to that of conventional maxizymes.

The present invention will be hereafter described in detail.

### (Maxizyme)

Maxizyme in the present invention means a minimized hammerhead ribozyme that functions as a dimer. This minimization is generally constructed by substituting the stem-loop II region of a hammerhead ribozyme which is composed of the antisense regions (stem I and stem II), the activity center region and the stem-loop II region, with a short chain linker. The maxizyme requires Mg²⁺ (magnesium ion) for the expression of its activity, and the GC base pairs in the stem II moiety are important to form a dimer. Design of the sequences of hammerhead ribozymes and maxizymes is also described in Biophysics, pp. 99-104, Vol. 40 No. 2 (2000), and all the contents of this publication are incorporated herein by reference as a part of the disclosure of the present specification.

The number of sites capable of forming a cavity for capturing magnesium ions, that is, the activity center regions, may be one or two per maxizyme. A maxizyme having two activity center regions shows higher substrate-cleaving efficiency, if it binds simultaneously at the two positions of one substrate and can cleave the substrate after the NUX triplets of these two positions (wherein N represents A, G, C or U, and X represents A, C or U).

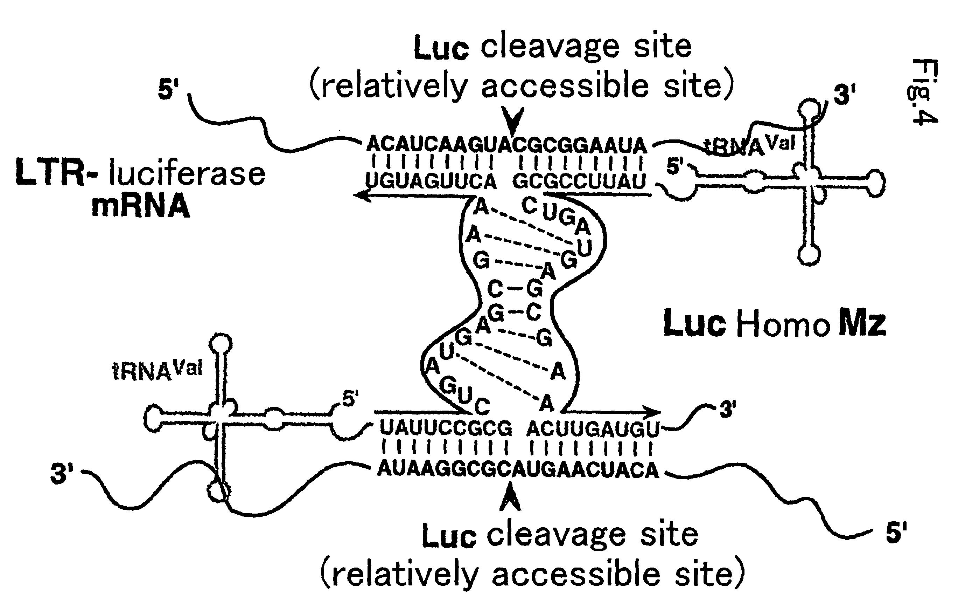

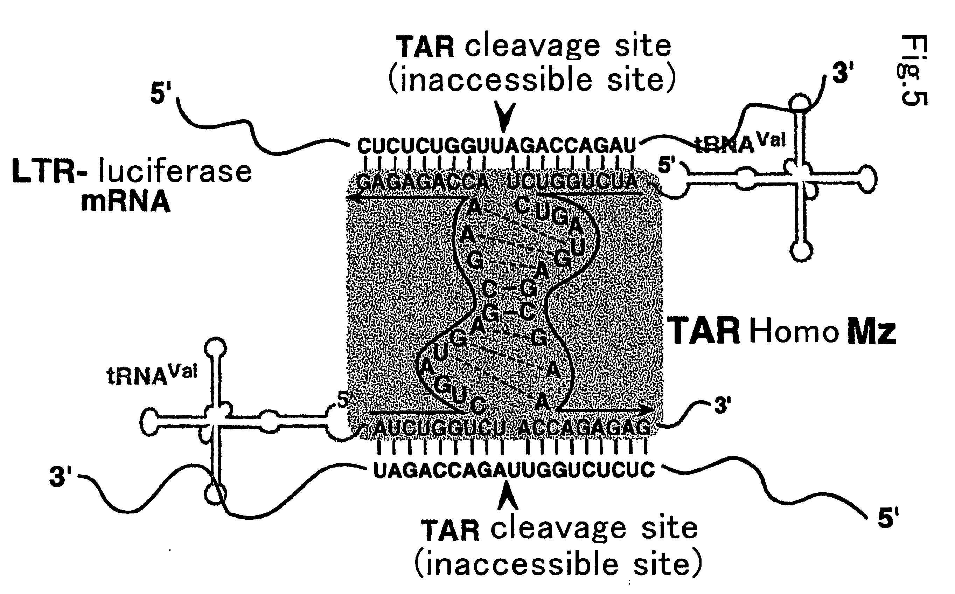

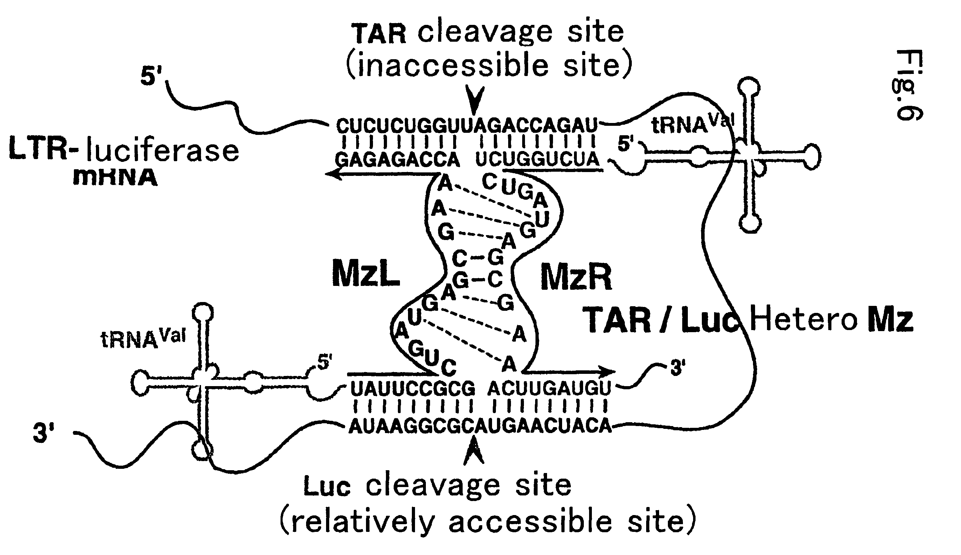

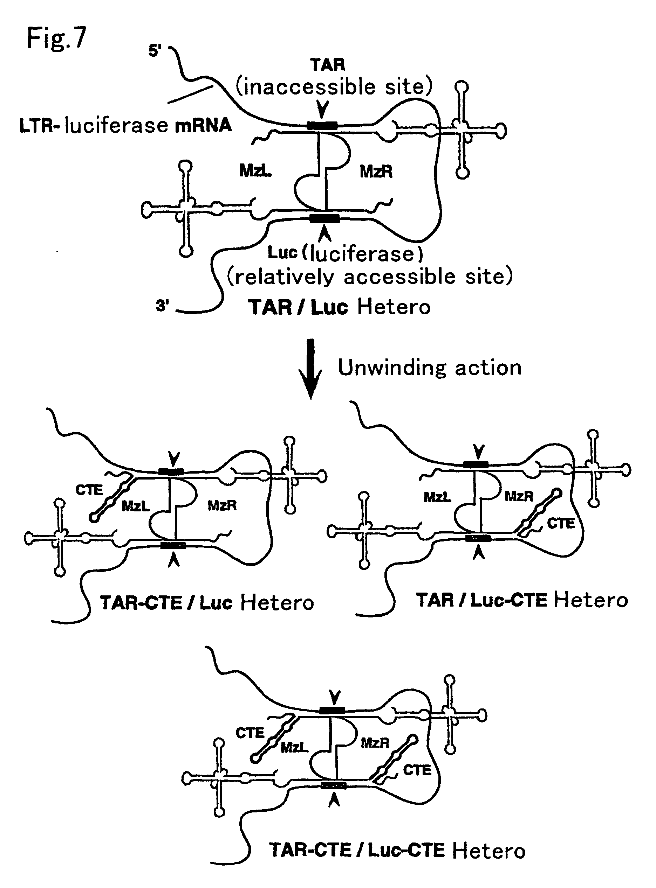

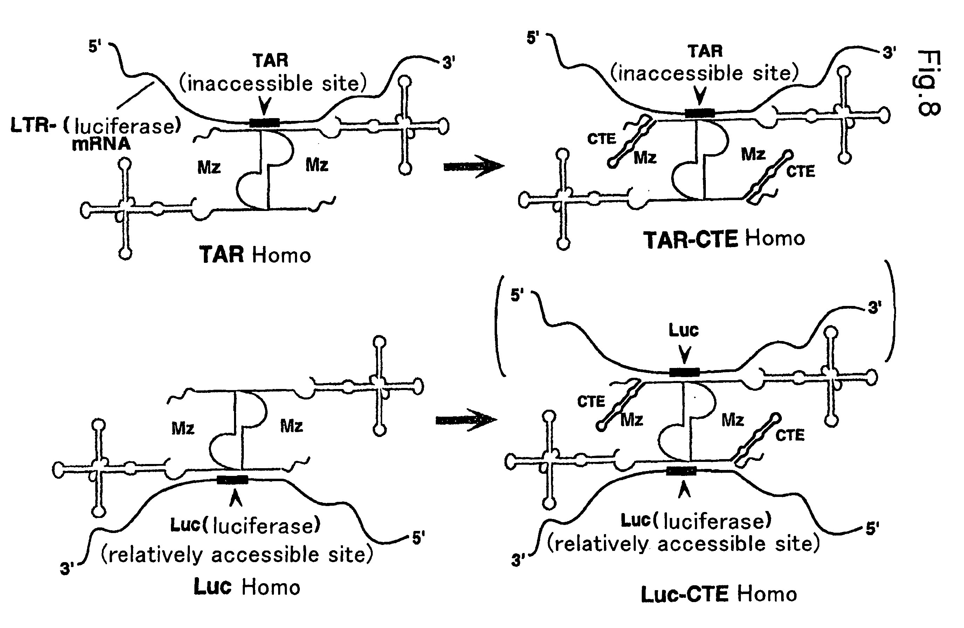

Because of its dimeric structure (dimer), the maxizyme binds to a target mRNA at two positions. Upon dimer formation, a homodimer type maxizyme (Figs. 4 and 5) wherein 2 monomers of 1 type are bound, or a heterodimeric maxizyme (Fig. 6) wherein 2 different types of monomers are bound, can be designed. The homodimer type maxizyme binds at its two positions to mRNAs having pre-determined binding sites. In this case, the two mRNAs bind to one maxizyme within the molecule (trans-type maxizyme, Fig. 7). In contrast, the heterodimer type maxizyme can bind to two different sequence portions. Specifically, the maxizyme binds simultaneously at the different positions within one mRNA (cis-type maxizyme, Fig. 8), or binds to two mRNAs wherein each of the different sequence portions individually binds at a position between the maxizyme (trans-type maxizyme) and a molecule (two mRNAs).

### (Molecule having helicase activity)

The molecule having helicase activity in the present invention means a molecule which acts on mRNA and makes the helical structure of the RNA unstable, so as to open the conformation containing double-stranded RNA. Specific examples of the molecule include an RNA helicase, ribosome, restriction enzyme (e.g., EcoRI and EcoRV) and the like. Such a molecule having helicase activity may directly bind to the maxizyme or bind via an adaptor molecule. The site for the molecule having helicase activity to bind to the maxizyme is not specifically limited, as long as it does not deteriorate the cleavage activity of the maxizyme. Preferably, the site is near the 3' end of the maxizyme.

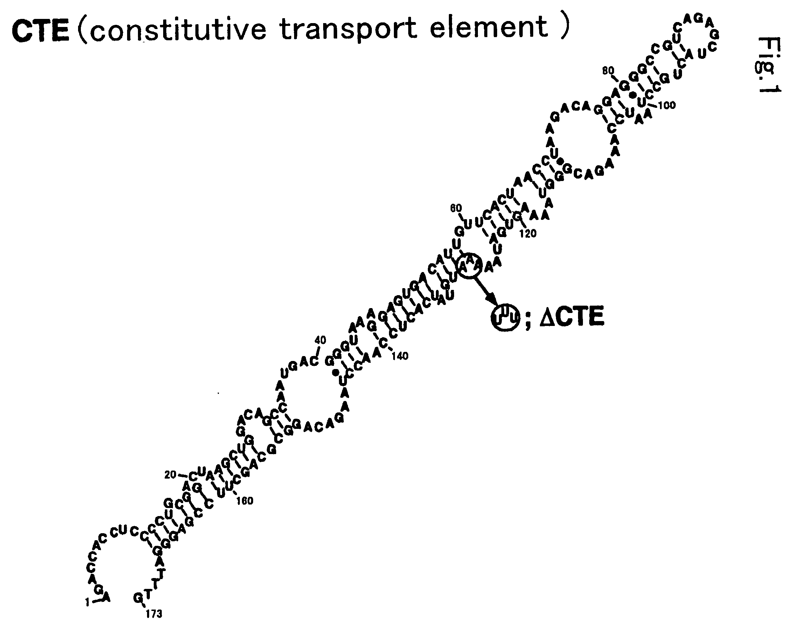

For allowing the molecule having helicase activity to bind to the maxizyme, there is a need to add a new binding region such that the molecule having helicase activity or the above adaptor molecule can stably bind to maxizyme. The binding region of the molecule or the like having helicase activity differs depending on a target molecule having helicase activity or an adaptor. It is possible to design such a binding region from the RNA double-stranded binding region of the molecule having helicase activity. For example, it is preferred to add a constitutive transport element (CTE, SEQ ID NO: 1, Fig. 1), HIV TAR RNA, POLY A RNA or the like as the binding region of the molecule having helicase activity.

CTE indicates a cis-acting virus RNA for transporting an unspliced genomic RNA to the cytoplasm in order to perform expression and packaging of a virus structural protein. CTE is an RNA that a monkey type D retrovirus such as Mason-Pfizer monkey virus (MPLV) originally possesses. It is considered that these viruses possess the CTE, an RNA motif, for the extranuclear transport of an unspliced RNA (H. Tang et al., (1997) Science 276: 1412-1415; J. Li et al., (1999) Proc. Natl. Acad. Sci. USA 96:709-714; H. Tang et al., (1999) Mol. Cell Biol. 19: 3540-3550).

In a preferred embodiment of the present invention, the above CTE, or an RNA having functions substantially equivalent to those of the CTE, or a mutant of the CTE sequence having functions substantially equivalent to those of the CTE can be used. Here, the expression "RNA having functions substantially equivalent to those of CTE" means a molecule other than CTE having binding affinity for RNA helicase. For example, it means a molecule such as an aptamer, which binds to helicase and is artificially produced by the SELEX method. In addition, the term "mutant" means a mutant wherein one or multiple nucleotides are altered (substituted, deleted, added or inserted). Such alteration can be performed by methods described in general publications such as J. Sambrook et al, Molecular Cloning A Laboratory Manual, Cold Spring Harbor Laboratory Press (1989).

PolyA RNA interacts with RNA helicase via the interaction between poly A-binding protein (PABP) and protein-1 that interacts with PABP.

The binding region of a molecule having helicase activity may be present in only one or both monomers of the maxizyme (homodimer or heterodimer) (Fig. 7). When the binding region is present in both monomers of the maxizyme, an extremely large steric hindrance is predicted. Even in this case, excellent mRNA-cleaving activity has been confirmed.

### (Maxizyme binding to a molecule having helicase activity)

Specific examples of the maxizyme of the present invention characterized by binding to a molecule having helicase activity or containing the region binding to a molecule having helicase activity include, but are not limited to, a maxizyme having binding affinity for helicase or a molecule that forms a complex with helicase, preferably, a maxizyme having binding affinity for RNA helicase or a molecule (adaptor) that forms a complex with RNA helicase, such as a maxizyme having an RNA sequence referred to as CTE (constitutive transport element), HIV TAR RNA sequence or POLY A RNA sequence, which binds with RNA helicase A.

The maxizyme of the present invention which binds to a molecule having helicase activity, normally comprises a nucleic acid sequence which has binding affinity for helicase or a molecule that forms a complex with helicase, and a nucleic acid sequence of the maxizyme, which is bound directly or indirectly to the former nucleic acid sequence. For example, the maxizyme can be chemically synthesized by using a DNA/RNA synthesizer (e.g., model 394, Applied Biosystems). As a method of binding both nucleic acid sequences, the nucleic acid sequence having binding affinity for helicase or the molecule that forms a complex with helicase may be located upstream or downstream of the nucleic acid sequence of maxizyme. Preferably, the nucleic acid sequence having binding affinity for helicase or the molecule that forms a complex with helicase is bound downstream of the nucleic acid sequence of the maxizyme. In such a case, the activity efficiency of the maxizyme is further improved.

### (Expression system of maxizyme)

In order to highly express the maxizyme of the present invention which binds to a molecule having helicase activity within cells, it is required that a promoter sequence is located upstream of the maxizyme sequence. When the expression system of pol III is used, tRNA^{val} sequence is added as an excessive sequence (promoter sequence other than the maxizyme portion) in this expression system. An expression vector of this maxizyme was actually constructed. In the construct which was expressed from this vector, the maxizyme sequence is bound downstream of tRNA^{val} sequence via a short chain linker.

As shown in Figs. 7 and 8, it was predicted that helicase bound to the 3' end and tRNA^{val} at the 5' end are extremely great steric hindrances for the maxizyme. However, a type of the maxizyme wherein the tRNA^{val} sequence had been added could be confirmed to have clear cleavage activity. Thus, it was confirmed that the maxizyme of the present invention could efficiently form a dimer.

### (Expression vector)

The expression vector encoding the maxizyme of the present invention which binds to a molecule having helicase activity can be constructed, for example, by incorporating a DNA obtained by ligating a promoter sequence, terminator sequence and the DNA sequence encoding the above maxizyme, into an appropriate vector.

Examples of the expression vector that can be used herein include plasmid vectors such as pUC19 (TAKARA SHUZO, Kyoto), pGREEN LANTERN (Life Tech Oriental, Tokyo), pHaMDR (HUMAN GENE THERAPY 6: 905-915 (July 1995)), and vectors for gene therapy such as adenovirus vectors and retrovirus vectors.

The above vector may contain a promoter sequence upstream of the DNA encoding the maxizyme. A promoter sequence is an element for controlling the expression of the DNA, and examples of the promoter include virus promoters (e.g., an SV40 promoter), phage promoters (e.g., a λPL promoter) and pol III promoters (e.g., a human tRNA promoter (e.g., a tRNA^{val} promoter) or adenovirus VA1 promoters). In the present invention, pol III promoter, and in particular tRNA promoter, can be preferably used.

The vector of the present invention may further contain a terminator sequence downstream of the DNA encoding the maxizyme that binds to a molecule having helicase activity. Any sequence can be used as the terminator, as long as it is a sequence that can terminate transcription. When necessary, the vector can contain a selection marker gene such as an antibiotic resistance gene (e.g., Amp^{r} and Neo^{r}) and a gene for complementing a nutritional requirement, or a reporter gene.

The nucleic acid encoding the maxizyme of the present invention may be chemically synthesized by using a DNA/RNA synthesizer, or can be obtained by synthesizing RNA in the presence of DNA-dependent RNA polymerase enzyme using the above expression vector DNA as a template, and then collecting the synthesized RNA. When introduced into a cell, the expression vector of the present invention is integrated on a chromosome by homologous recombination, and then the maxizyme of the present invention which binds to a molecule having helicase activity can be expressed. For allowing homologous recombination to occur, DNA encoding the maxizyme of the present invention which can bind to a molecule having helicase activity is inserted into a sequence homologous to a part of a host cell genome, and the thus obtained product can be incorporated into a vector DNA. Integration onto a chromosome can be performed using not only an adenovirus vector, retrovirus vector or the like that is used in a gene therapy, but also a plasmid vector.

### (Complex of the maxizyme of the present invention and a molecule having helicase activity)

The present invention further encompasses a complex of the maxizyme characterized by binding to a molecule having helicase activity and a molecule having helicase activity. Specific examples of a molecule having helicase activity include an RNA helicase, ribosome, and restriction enzyme (e.g., EcoRI and EcoRV). The RNA helicase is preferred. Binding of the maxizyme to the molecule having helicase activity may be achieved via either covalent binding or non-covalent binding, as long as it does not deteriorate the function of each component. In the case of non-covalent binding, the molecule can bind to the CTE or polyA sequence via an adaptor that binds specifically to helicase. In the case of covalent binding, the molecule can bind to the maxizyme via a linker, if necessary.

### (Pharmaceutical composition)

The present invention further encompasses a pharmaceutical composition comprising the aforementioned maxizyme, expression vector or complex as an active ingredient. The pharmaceutical composition of the present invention can contain a pharmaceutically acceptable carrier (for example, a diluent such as physiological saline or buffer), if necessary. Application of the pharmaceutical composition of the present invention depends on the types of the function of the maxizyme. Namely, the pharmaceutical composition of the present invention can be used to prevent or treat diseases caused by a target RNA of the maxizyme.

For example, the pharmaceutical composition of the present invention is useful for preventing or treating diseases caused by viruses such as the AIDS virus (HIV), hepatitis type C virus or hepatitis type B virus, apoptosis-related diseases (e.g., Alzheimer's disease and Parkinson's disease), cancer, autoimmune disease, inflammation, genetic diseases, or the like.

The maxizyme used in the present invention cleaves the nucleic acids which is a causative substance of a disease, binds complementarily to such a nucleic acid so as to inhibit the functions, or specifically binds to a pathogenic protein so as to inhibit the functions. Thus, the maxizyme can deteriorate the normal functions of the causative substance.

Examples of a method for introducing the maxizyme of the present invention or the vector containing the DNA encoding such maxizyme into a cell include the calcium phosphate method, the electroporation method, the lipofection method, the microinjection method, the method using a gene gun, and the method using liposomes (e.g., Mamoru NAKANISHI et al., Protein Nucleic Acid Enzyme Vol. 44, No. 11, 1590-1596 (1999)). When the vector is used, the vector can be introduced into a cell by the above method. For example, a part of the cells of a disease locus is taken out, gene introduction is performed in vitro, and then the cells can be returned into the tissue. Alternatively, the vector can be directly introduced into the tissue of an affected portion. In the case of infection by virus vectors, the virus titer is normally at approximately 10⁷ pfu/ml or more.

### (Use of the maxizyme, expression vector or complex of the present invention)

The present invention further provides a method for specifically cleaving a target nucleic acid, or inhibiting or suppressing the biological functions of a target nucleic acid by using the maxizyme, expression vector or complex of the present invention. For example, this method can also be used for elucidating the biological functions of a target nucleic acid. Randomization of the sequences of the target binding sites (stem I and stem III) of the maxizyme enables elucidation of a gene necessary for a certain biological function.

Specifically, the maxizyme having the randomized target binding sites as mentioned above is introduced into a cell. For example, introduction of the maxizyme into normal cells can induce canceration, or introduction of the same into abnormal cancer cells can result in the recovery to normal cells. These procedures can lead to the elucidation of a gene involved in canceration. When necessary, the sequence of the gene is examined using GenBank or the like, so that the entire sequence and the functions of the gene can be elucidated. When the gene is an unknown gene, the entire sequence can be determined by cloning the target gene based on the sequence of the target binding site. Even when the above random sequence is complementary to that of an important gene, most of the random sequences fail to cleave the target because they are unable to interact with the target due to the higher-order structures of the targets. In the present invention, by using the maxizyme which can bind to a molecule having helicase activity, the above problems can be avoided and thus the efficiency can be greatly improved.

All of the contents disclosed in the specification of Japanese Patent Application No. 2001-134469, which is a priority document of the present application, is incorporated herein by reference as a part of the disclosure of the present application.

The present invention will be described more specifically by the following examples, but the present invention is not limited by these examples.

### EXAMPLE

In these examples, intracellular activity of the maxizyme having a nucleotide sequence that binds to a molecule having helicase activity on the target mRNA was evaluated. In these examples, RNA helicase A was selected as a molecule having helicase activity, and a maxizyme (CTE-Mz) wherein CTE RNA as a motif to which this molecule can bind had been ligated downstream of the maxizyme, was used.

### Example 1: Construction of maxizyme expression vector (CTE-Mz expression vector) having a nucleotide sequence that binds to a protein having helicase activity

In order to prepare the maxizyme of the present invention, the sequence of the maxizyme was determined so that the target sites of the maxizyme would be a TAR RNA region (which is proven to have a strong secondary structure) within an HIV-1-derived Long Terminal Repeat (LTR) gene and luciferase mRNA (which allows quantitative evaluation).

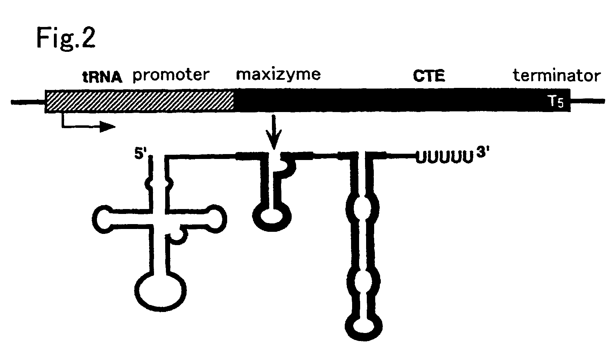

A CTE-Mz expression vector was constructed according to the method described in a publication (J. Virol. 73, 1868-1877 (1999); Proc. Natl. Acad. Sci. USA 96, 1886-1891 (1999)). A single-stranded oligonucleotide was chemically synthesized which has restriction sites Csp45I and SalI at the 5' and 3' ends, respectively, the DNA sequence encoding the maxizyme (SEQ ID NOS. 4, 5, 6 and 7) from upstream in the middle region, restriction sites KpnI and EcoRV, and a terminator sequence (TTTTT) at the 3' end. Polymerase chain reaction was carried out using the chemically synthesized oligonucleotide as a template to synthesize a double-stranded oliogonucleotide. This double-stranded oligonucleotide was treated with restriction enzymes Csp45I and SalI, and then the digest was inserted downstream of tRNA^{val} of a plasmid pUC-dt that had been similarly treated with restriction enzymes Csp45I and SalI. The product was treated with restriction enzymes KpnI and EcoRV, and then a DNA sequence encoding a constitutive transport element (CTE, Fig. 1) derived from a Simian type D retrovirus (SRV) gene was inserted, thereby obtaining a CTE-Mz expression vector (Fig. 2).

### Example 2: Evaluation of Mz activity with luciferase activity

HeLa cells (LTR-Luc HeLa) which stably expresses a chimeric gene (SEQ ID NO: 8) comprising a Long terminal repeat (LTR) gene ligated to a luciferase (Luc) gene, were used for the evaluation. 1x10⁵ cells of LTR-Luc HeLa were inoculated on a 12-well plate, and then cultured overnight at 37°C in a 5% carbon dioxide (gas) incubator. 2 µg each of CTE-Mz expression vectors Mz L and Mz R (in the case of homomaxizyme, 4 µg of monomer maxizyme) obtained in Example 1, 100 ng of HIV-1-derived Tat gene expression plasmid (Tat protein is essential for LTR-Luc expression), and 50 ng of pSV β-galactosidase (Promega) were added to an Opti mem I medium (GIBCO/BRL) containing 4 µl of a Lipofectin agent (GIBCO/BRL, Rockville, MD, U.S.A.), followed by incubation at room temperature for 30 minutes or more. Then, the mixture was dropped onto the cells in the 12-well plate, thereby performing transfection. After 24 hours of culturing at 37°C in a carbon dioxide (gas) incubator, the cells were collected, and then the luciferase activity was measured using a PicaGene kit (TOKYO PRINTNG INK MFG. Co., LTD., Tokyo, Japan). The cells were lysed in 150 µl of a cell lysis solution (100 mM K₂HPO₄, 100 mM KH₂PO₄, 0.2% Triton X-100 and 1 mM DTT; pH7.8), and then centrifuged at 10,000 x g at 4°C for 1 minute. 10 µl of the supernatant was added to 100 µl of a luciferase activity measurement reagent (20 mM Tricine, 1.07 mM (MgCO₃)₄Mg(OH)₂, 2.67 mM MgSO₄, 0.1 mM EDTA, 33.3 mM DTT, 270 µM coenzyme A, 470 µM luciferin and 530 µM ATP). 3 seconds after the addition, the fluorescent intensity was measured for 10 seconds using a fluorometer (Lumant LB 9501, Berthold, Bad Wildbad, Germany). The effect of the transfection efficiency on luciferase activity was corrected with β-galactosidase activity derived from the pSV β-galactosidase which had been simultaneously transfected.

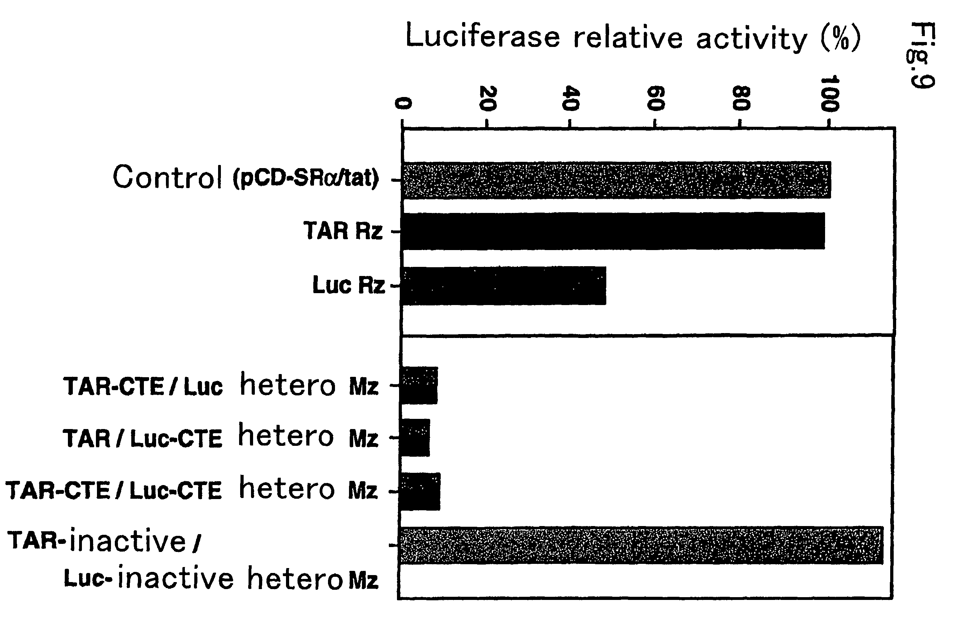

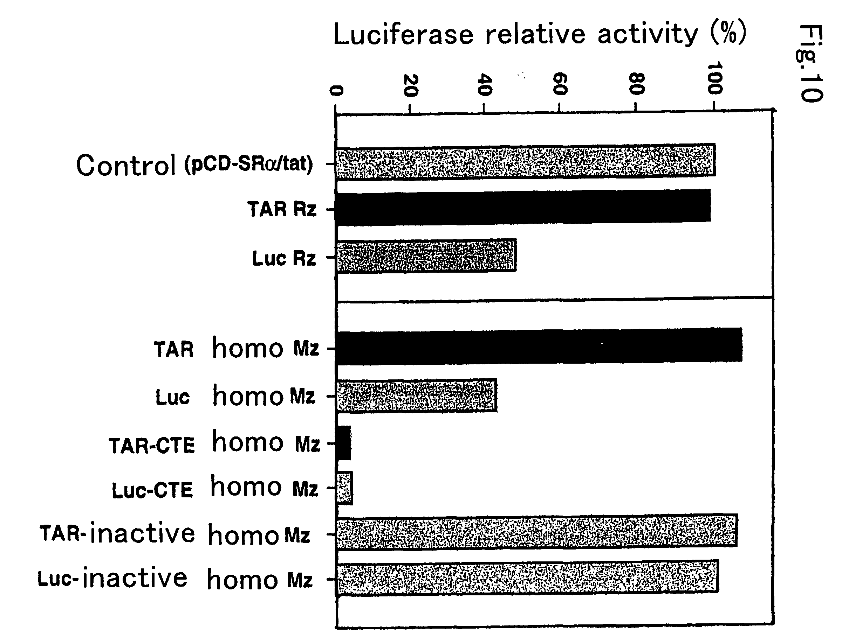

These results are shown in Figs. 9 and 10. Luciferase activity when a control (pCD-SRα/tat) was allowed to act is determined as 100%, and the relative activities against it were shown.

When CTE-Mz was allowed to act on LTR-luciferase mRNA, significantly low luciferase activity was shown as compared with a case when a normal Mz was allowed to act. Thus, it was revealed that CTE-Mz showed excellent cleavage activity also for TAR and Luc that are cleaved with difficulty with the normal Mz.

### Industrial Applicability

The present invention makes it possible to provide a maxizyme which binds to a target mRNA regardless of its conformation, and can effectively cleave it. The maxizyme of the present invention is useful as a medicament. Furthermore, the maxizyme is also useful in a method for specifically cleaving a target nucleic acid and a method for inhibiting or suppressing the biological functions of a target nucleic acid. By using such methods, the biological functions of a target nucleic acid can also be elucidated.

## Claims

1. A maxizyme which binds to a molecule having helicase activity.

2. A maxizyme which comprises a region which binds to the molecule having helicase activity.

3. A maxizyme which is composed of a dimeric structure formed by an RNA molecule containing the following nucleotide sequence (a) and an RNA molecule containing the following nucleotide sequence (b),
5' X¹ ₗ...X¹ ₕ Y¹ ₗ...Y¹ ᵢZ¹ ₗ...Z¹ ⱼB¹ ₗ...B¹ ₚ 3' (a)
5' Z² ₗ...Z² ₙ Y² ₗ...Y² ₘX² ₗ...X² ₖB² ₗ...B² _{q} 3' (b)
wherein X¹ₗ...X¹ₕ, X²ₗ...X²ₖ, Y¹ₗ...Y¹ᵢ, Y²ₗ...Y²ₘ, Z¹ₗ...Z¹ⱼ, Z²ₗ...Z²ₙ, B¹ₗ...B¹ₚ and B²ₗ...B²_{q} are independently any one of A, U, T, C and G; and h and k are integers of 1 or more (for example, integers between 1 and 100);
i and m are integers of 1 or more (for example, integers between 1 and 100);
j and n are integers of 1 or more (for example, integers between 1 and 100);
p and q are integers of 1 or more (for example, integers between 1 and 100);
X¹ₗ...X¹ₕ and X²ₗ...X²ₖ are nucleotide sequences complementary to a specific sequence in a target RNA, or nucleotide sequences containing a region complementary to a sequence near a cleavage site of the target RNA and a region capable of forming a cavity for capturing Mg²⁺ (magnesium ion) only in the presence of the target RNA;
Y¹ₗ...Y¹ᵢ and Y²ₗ...Y²ₘ are nucleotide sequences forming stems; and
Z¹ₗ...Z¹ⱼ and Z²ₗ...Z²ₙ are nucleotide sequences containing a region complementary to a sequence near a cleavage site of the target RNA and a region capable of forming a cavity for capturing Mg²⁺ only in the presence of the target RNA, and one of or both B¹ₗ...B¹ₚ and B²ₗ...B²_{q} are nucleotide sequences containing a region that binds to a molecule having helicase activity.

4. The maxizyme according to claim 3, wherein, as a nucleotide sequence capable of forming a cavity for capturing Mg²⁺ (magnesium ion) only in the presence of a target RNA, Z¹ₗ...Z¹ⱼ contains a sequence GAA, and Z²ₗ...Z²ₙ contains a sequence CUGAY_{Z}GA wherein Y_{Z} represents any one mononucleotide of A, G, U or C.

5. The maxizyme according to claim 3 or 4, which contains, as a nucleotide sequence capable of forming a cavity for capturing Mg²⁺ only in the presence of a target RNA, a sequence GAA of X²ₗ...X²ₖ and a sequence CUGAY_{X}GA of X¹ₗ...X¹ₕ wherein Y_{X} represents any one mononucleotide of A, G, U or C.

6. The maxizyme according to any one of claims 1 to 5, wherein the molecule having helicase activity is an RNA helicase.

7. The maxizyme according to claim 6, wherein the region that binds to a molecule having helicase activity is nucleotides represented by SEQ ID NO: 1 (CTE).

8. The maxizyme according to claim 6, wherein the region that binds to a molecule having helicase activity is nucleotides represented by SEQ ID NO: 2 (TAR).

9. The maxizyme according to any one of claims 3 to 8, wherein a linker sequence and a tRNA^{val} promoter sequence are added upstream of each of the nucleotide sequences (a) and (b).

10. The maxizyme according to claim 9, wherein the linker sequence added upstream of the nucleotide sequence (a) contains the following nucleotide sequence (e), and the linker sequence added upstream of the nucleotide sequence (b) contains the following nucleotide sequence (f):
5' AAA 3' (e)
5' UUU 3' (f)

11. The maxizyme according to claim 9, wherein the tRNA^{val} promoter sequence added upstream of each of the nucleotide sequences (a) and (b) is SEQ ID NO: 3.

12. The maxizyme according to claim 9, wherein an additional sequence and a terminator sequence are added downstream of each of the nucleotide sequences (a) and (b).

13. The maxizyme according to claim 12, wherein the additional sequence added downstream of the nucleotide sequence (a) contains the following nucleotide sequence (g), the additional sequence added downstream of the nucleotide sequence (b) contains the following nucleotide sequence (h), and the terminator sequence added downstream of each of the nucleotide sequences (a) and (b) contains the following nucleotide sequence (i):
5' AAA 3' (g)
5' AACCGUA 3' (h)
5' UUUUU 3' (i)

14. An expression vector which contains a DNA encoding the maxizyme according to any one of claims 1 to 13.

15. A complex which is composed of the maxizyme according to any one of claims 1 to 13 and a molecule having helicase activity.

16. A pharmaceutical composition, which comprise the maxizyme according to any one of claims 1 to 13, the expression vector according to claim 14, or the complex according to claim 15 as an active ingredient.

17. A method for cleaving a target nucleic acid by using the maxizyme according to any one of claims 1 to 13, the expression vector according to claim 14, or the complex according to claim 15.

18. A method for specifically inhibiting or suppressing the biological functions of a target nucleic acid by using the maxizyme according to any one of claims 1 to 13, the expression vector according to claim 14, or the complex according to claim 15.

19. A method for analyzing the biological functions of a target nucleic acid, which comprises specifically cleaving the target nucleic acid or specifically inhibiting the biological functions of the target nucleic acid by using the maxizyme according to any one of claims 1 to 13, the expression vector according to claim 14, or the complex according to claim 15; and examining the effect of the cleavage or the inhibition on the biological activities.
